Europäisches Patentamt

European Patent Office

Office européen des brevets

(11) Publication number: **0 353 908 B1**

## EUROPEAN PATENT SPECIFICATION

(45) Date of publication of patent specification: **17.05.95**  (51) Int. Cl.⁶: **C12N 15/29**, C12N 15/32, C12N 15/82

(21) Application number: **89307342.9**

(22) Date of filing: **19.07.89**

(54) **DNA constructs and plant incorporating them.**

(30) Priority: **05.08.88 GB 8818706**

(43) Date of publication of application:
**07.02.90 Bulletin 90/06**

(45) Publication of the grant of the patent:
**17.05.95 Bulletin 95/20**

(84) Designated Contracting States:
**AT BE CH DE ES FR GB GR IT LI LU NL SE**

(56) References cited:
**EP-A- 0 193 259**
**EP-A- 0 240 208**
**EP-A- 0 256 223**

**MGG MOLECULAR AND GENERAL GENETICS**
**vol. 215, no. 3, February 1988 pp. 431-440,**
**Berlin, DE; T.D. SULLIVAN et al.**

**Nature, vol. 323, October 1986, pp. 551-554,**
**London, GB; J. SIMPSONet al.**

**NATURE, vol. 316, August 1985, pp. 750-752,**
**London, GB; G. LAMPPA et al.**

(73) Proprietor: **ZENECA LIMITED**
**15 Stanhope Gate**
**London W1Y 6LN (GB)**

(72) Inventor: **Ely, Susan**
**64 Newfield Gardens**
**Marlow**
**Bucks S17 1JP (GB)**
Inventor: **Ray, John Anthony**
**30 Sylvanus**
**Wooden Hill**
**Bracknell RG12 4XX (GB)**
Inventor: **Schuch, Wolfgang Walter**
**14 Greenfinch Close**
**Heathlake Park**
**Growthorne**
**Berkshire (GB)**
Inventor: **Knight, Mary Elizabeth**
**14 Greenfinch Close**
**Heathlake Park**
**Growthorne**
**Berkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

THE EMBO JOURNAL, vol. 6, no. 9, 1987, pp.
2537-2542, Oxford, GB; F. NAGY et al.

NATURE

(74) Representative: **Huskisson, Frank Mackie et al**
**Intellectual Property Group,**
**Zeneca Seeds,**
**Jealott's Hill Research Station,**
**PO Box No 3539**
**Bracknell,**
**Berkshire RG12 6YD (GB)**

**Description**

The present invention relates to DNA constructs and to plants incorporating them.

Manipulation of crop plants for improved productivity requires the expression of foreign or endogenous genes in plant tissues.

Constitutive expression of genes may be required in many situations. However, the restriction to particular tissue types may also be needed. For example, toxin genes are best not expressed in the endosperm of cereal plants, as these are the parts of the plants used for feed.

The present invention relates to a green-tissue-specific expression cassette for the expression of exogenous genes in plants.

When photosynthetic plants are exposed to light a number of important events take place. Among these are the reorganisation of membranes in the chloroplast, the activation of enzymes present there, and the activation of nuclear genes responsible for the synthesis of polypeptides capable of light harvesting and light-driven electron transport. One of these proteins is the chlorophyll a/b binding protein (CAB) associated with the photosystem II of the thylakoid membranes (1-4). The CAB mRNA and polypeptides are increased after illumination of dark grown maize plants. The CAB mRNA can reach levels in greening young leaves as high as 0.5-1% of mRNA in the cell (5).

Accordingly we propose the maize CAB gene as a useful source for the isolation of green-tissue specific promoters, which, we expect, will allow a high level of expression of foreign proteins, particularly in maize.

CAB mRNAs and genes have been cloned from a number of sources, including wheat (mRNA and genes) (6), pea (mRNA and genes) (7), cucumber (mRNA) (8), corn (mRNA) (4), tomato (mRNA and genes) (9) and tobacco (10). It has been demonstrated that the promoters of the wheat CAB gene whAB 1.6 (11) and the pea CAB gene AB 80 (12) are capable of expressing a bacterial marker gene in transgenic tobacco. Within the promoter fragment of 1.8 kb (whAB 1.6) a shorter sequence of 268 basepairs has been delineated which allows the light regulated expression of the bacterial marker gene (12). The same sequence has been demonstrated in the pea gene (13). Within this sequence is also found an element which "silences" the expression of this promoter in roots (13).

We have isolated a CAB gene from corn (Zea mays) so that the promoter of this gene can be used to express exogenous genes in corn and other plants: such as wheat and barley, and dicots such as tobacco, tomato, potato and sugar-beet. The maize CAB promoter is capable of expressing a wide variety of exogenous genes: including in particular the δ-endotoxin gene from B. thuringiensis in transgenic plants, leading to protection of these plants from insect attack.

Accordingly, the present invention provides a DNA construct comprising the promoter from a chlorophyll a/b binding (CAB) protein gene derived from the plant Zea mays, said promoter being operatively linked to DNA coding for RNA exogenous to Zea mays, whereby the construct expresses the exogenous RNA only under control of the promoter.

The invention further provides vectors containing such DNA constructs: such vectors may for example be bacteriophages or plasmids.

The invention further provides plant genomes having stably incorporated therein recombinant DNA comprising the promoter from a chlorophyll a/b binding protein gene derived from the plant Zea mays, operatively linked to DNA coding for RNA exogenous to Zea mays, whereby the RNA is expressed only under control of the promoter.

We further provide plants having stably incorporated in their genome recombinant DNA comprising the promoter from a chlorophyll a/b binding protein gene, operatively linked to DNA coding for the expression of RNA exogenous to Zea mays, whereby the RNA is expressed only in plant green tissue.

The exogenous RNA produced by plants according to our invention may code for a functional protein, eg. an exogenous protein, for example an insecticidal protein such the δ-endotoxin of Bacillus thuringiensis. Alternatively, it may have some other function in the plant cell. For example, it may be anti-sense RNA, that is RNA which is complementary (in base-pairing and sequence orientation) to RNA naturally produced by the plant cell. Such antisense RNA is found to inhibit selectively the translation of the naturally-occurring RNA into protein. In this way, the invention provides a means, not only of promoting the formation of foreign proteins in green plant tissue, but also of reducing or inhibiting the formation of proteins naturally present. Examples of foreign proteins which may be formed in green plant tissue include enzymes which confer protection against attack by external agents, including herbicides, fungi and insects. For example, green tissue may be made resistant to herbicides such as glyphosate by incorporation of a resistant AroA gene, or to herbicides such as imidazoline and sulphonamide herbicides by incorporating a resistant acetolactate synthase (ALS) gene.

It is particularly useful to be able to promote the formation of proteins in green plant tissue that render such tissue resistant to attack by insects. Particularly useful proteins for this purpose are thionins, chitinases, protease inhibitors and the δ-endotoxin of Bacillus thuringiensis.

Examples of naturally present proteins where formation may be reduced or restricted are numerous. Examples of plants into which the constructs of our invention may be incorporated include both monocots and dicots. Currently, although several methods for transforming monocotyledonous plants have been reported success has been limited. Nevertheless, it seems inevitable that such techniques will soon become available, and accordingly it will be possible to incorporate the constructs of our invention into many different agronomically important monocot plants, including for example wheat, barley, maize, rye, oats, sugar cane, sorghum, alfalfa, forage grasses and rice. Maize is particularly preferred.

General methods for transforming dicots (generally based on the use of Agrobacterium tumefaciens or material derived from this micro-organism) are currently available, and may be used to produce plants according to our invention by transforming, for example, tobacco, potato, tomato, beet and other Brassica species such as cabbage and oilseed rape, cotton, peanuts, soya and sunflowers.

Examples of the invention, and experiments related thereto, will now be specifically described with reference to the drawings, in which :

Figure 1 illustrates schematically restriction maps for the DNA constructs gCAB48, pCAB48 and pCAB48.1.

Figure 2 gives the base sequence of the pCAB48.1 XbaI/NcoI promoter fragment.

Figure 3 shows the structure of the CAB gene in pCAB48.1.

Figure 4 outlines diagramatically the construction of the plasmids pCG1 and pCG2 from pCAB 48.1 and pTAK3.

Figure 5 shows the base sequence of the synthetic oligonucleotide used in the construction of plasmid pOL1.

Figure 6 outlines diagramatically the formation of plasmids pCBT1 and pCBT2.

EXAMPLE 1 - Isolation of gCAB 48

1.1 Construction of a maize genomic library

Total DNA was extracted from whole plants of greenhouse grown maize line BE10 (proprietary homozygous maize line ex Holdens). The method of Murray and Thompson (14) was used for the extraction of DNA. DNA was purified and partially restricted with restriction enzyme Sau3A as described by Maniatis et al (15). DNA fragments of approximately 20 kb were isolated from sucrose gradients, and cloned into the phage vector EMBL3, which had been cut with BamHI. The genomic library was plated onto K803 bacterial cells under standard conditions (Maniatis et al).

1.2 Screening of the genomic library

The genomic library ($10^5$ plaques) was screened using synthetic oligonucleotides corresponding to the published wheat CAB gene sequence (positions amino acid 56-63 and 202-207 of the mature CAB protein), and the cucumber CAB cDNA (8).

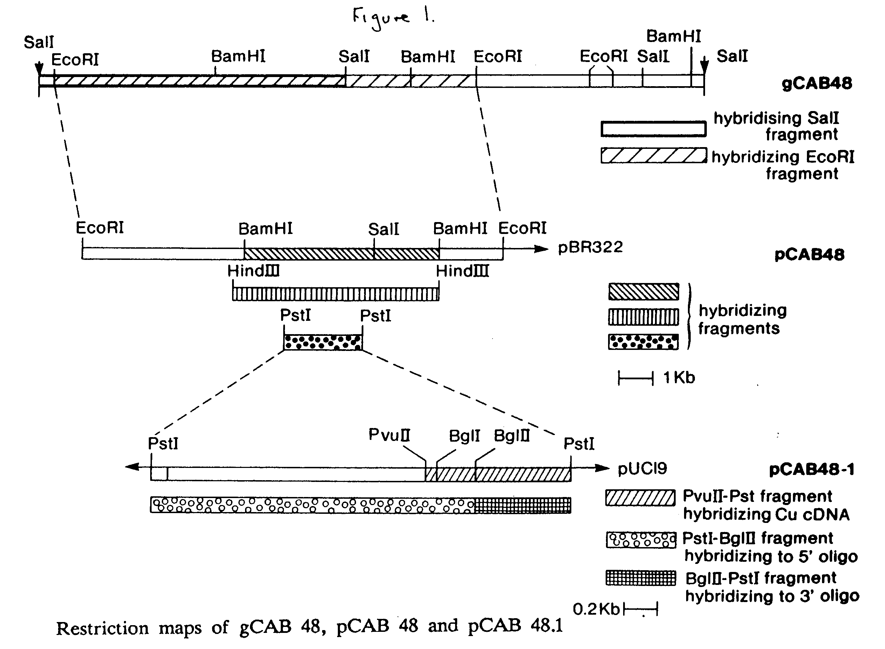

Hybridisation conditions for oligonulceotides were: 2XSSC, 42°C, with washes in 2XSSC at 42°C. Cucumber CAB cDNA was hybridised under the following conditions: 2XSSC, 65°C. After several rounds of hybridisation and plaque purification, one clone of about 20 kb was isolated which hybridised to both wheat oligonucleotides and the cucumber CAB cDNA. This clone was called gCAB 48. A map of this clone is shown in Figure 1 in which also shows subclones generated for further analysis. Referring further to Figure 1 pCAB48 is the plasmid obtained by cloning the 13 kb EcoRI fragment of gCAB48 into the publicly available plasmid pBR322. pCAB48.1 is the plasmid obtained by cloning the 2.8 kb PstI fragment of gCAB48 into the publicly available plasmid pUC19.

1.3 Sequence analysis of the gene encoded in gCAB 48

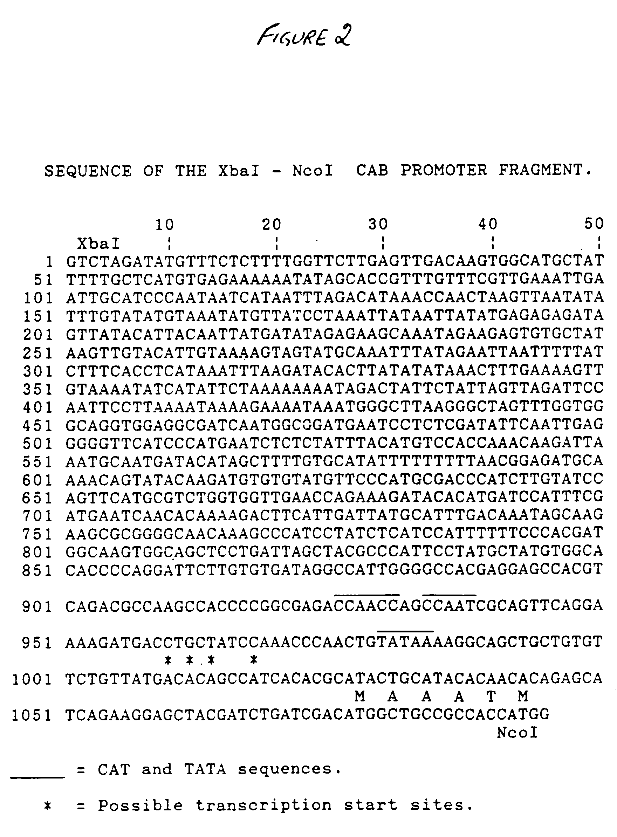

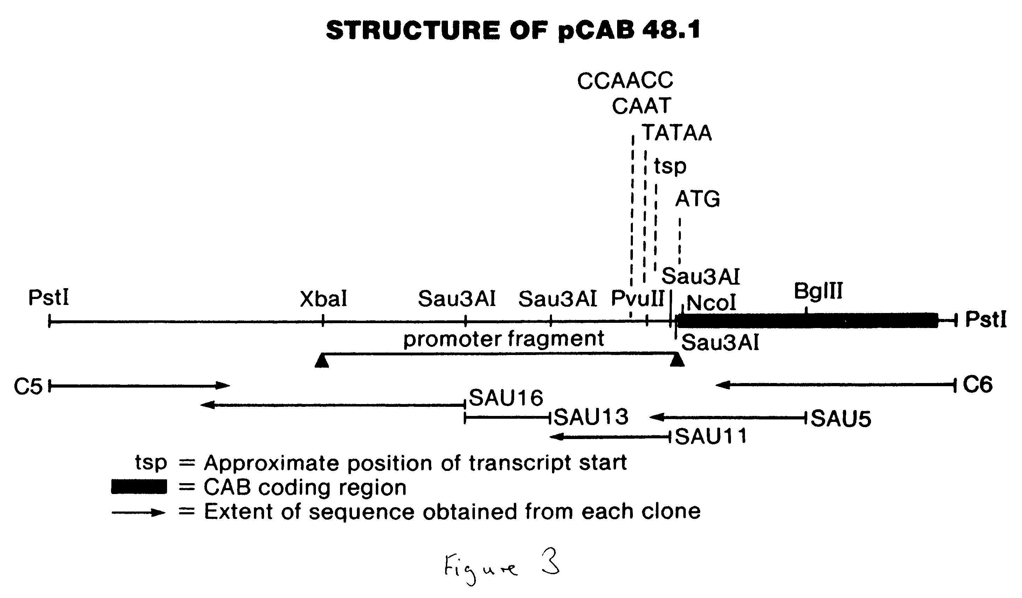

DNA was prepared from gCAB 48, and the hybridising Pst1 fragment of 2.8 kb subcloned into M13 for sequence analysis. This was carried out by the method of Sanger et al (16). The complete sequence of this gene and its 5′ and 3′ flanking regions contained on the PstI fragment was determined. Figure 2 shows the base sequence of the XbaI/NcoI BAB promoter fragment. Figure 3 shows the schematic representation of the CAB gene encoded in pCAB48.1. This gene encodes a complete CAB gene of 264 amino acids

4

including a leader sequence of 32 amino acids required for targeting to the chloroplast membranes. It also contains 5′ upstream sequences of 1.9 kb sufficient for the green-tissue specific expression required.

EXAMPLE 2 - Use of the CAB promoter to drive green tissue specific expression of a foreign gene in transgenic plants.

2.1 Isolation of the CAB promoter for translational fusions.

The 2.8 kb PstI fragment from 1.3 above containing the complete CAB gene was subcloned into the common cloning vector pUC19 to yield vector pCAB 48.1. The CAB promoter can be conveniently isolated from this plasmid on a XbaI-NcoI fragment. This piece of DNA contains 1.0 kb of 5′ upstream region (promoter), 60 bases of the 5′ untranslated region of the CAB mRNA, and 18 bases coding for amino acids of the pre-CAB polypeptide sequence. This promoter fragment is useful for the construction of translational fusions.

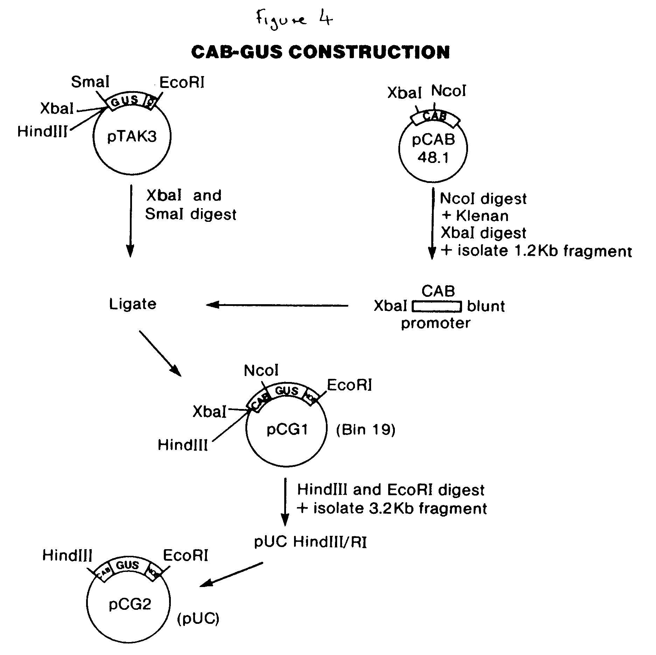

2.2 Fusion of the CAB promoter to the bacterial GUS gene (see Figure 4).

The translational fusion of the CAB promoter to the bacterial $\beta$-glucuronidase (GUS) gene was accomplished by cutting pCAB 48.1 with NcoI and filling in the ends under standard conditions using DNA polymerase Klenow fragment A. Then the plasmid was cut with XbaI. The 1.2 kb promoter fragment was isolated after separation on agarose gel electrophoresis, and purified by phenol extraction and ethanol precipitation. The fragment was then cloned into pTAK3 (17), cut with XhoI and SmaI. This procedure yielded plasmid pCG1, a binary plant transformation vector. The CAB-GUS expression cassette was easily transferred to publicly available plasmid pUC18 by restriction with HindIII and EcoRI as shown in Figure 4. The resulting plasmid was named pCG2.

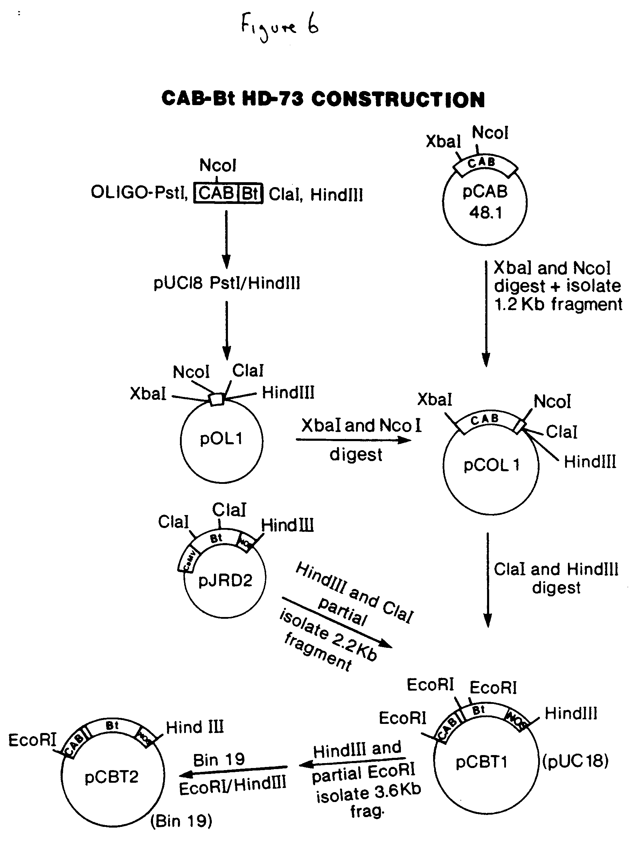

2.3 Translational fusion of the CAB promoter to the $\delta$-endotoxin gene from B. thuringiensis HD73 (see Figure 6).

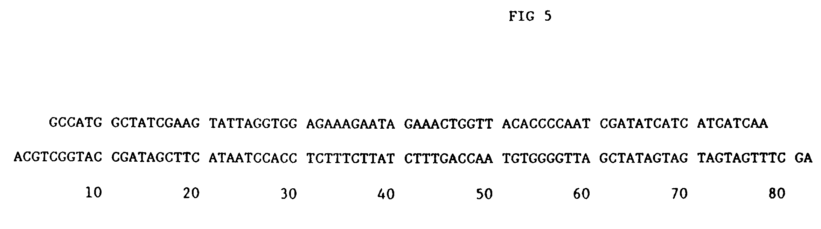

The CAB promoter can be used to drive the expression of an insecticidal gene such as the $\delta$-endotoxin gene from B. thuringiensis (B.t.). This is exemplified here by the fusion of the gene isolated from strain HD73 (obtainable from the US Department of Agriculture). However, any other B. thuringiensis gene may be used instead. An intermediary vector pOL1 was constructed by cloning a synthetic oligonucleotide of the structure shown in Figure 5 into pUC18. The truncated B.t. gene from pJRD2 (see below) was cloned into this vector. This is shown in Figure 6. Plasmid pJRD2 was constructed by excising the N-terminal portion of the $\delta$-endotoxin gene on a Dra1 fragment of 2.2 kb, and cloning this fragment behind the CaMV35S promoter in plasmid pJR1 (20). Plasmids including this fragment have been shown to confer insecticidal properties on bacterial strains and plants derived from them.

pOL1 was then digested with XbaI and NcoI and the 1.1 kb XbaI/NcoI promoter fragment from pCAB48.1 cloned into it. This gave the plasmid pCOL1. From this was generated the plasmid pCBT1 by digesting with ClaI and Hind III, and cloning in the 2.2 kb fragment isolated from pJRD2 by partial digestion with Hind III and ClaI. From pCBT1 was subsequently prepared pCBT2, by isolating the 3.6 kb CAB-Bt-nos fragment following digestion with Hind III and EcoRI (partial) and cloning into Bin 19.

2.4 Isolation of the CAB promoter for transcriptional fusions.

In a number of circumstances the CAB promoter may be required to drive the expression of a foreign gene in a transcriptional fusion. The promoter fragment can be isolated on a XbaI-Sau3A fragment after Xba1 and partial Sau3A restriction. The Sau3A sites are located 12 and 7 bases from the methionine start codon.

2.5 Fusion of the CAB promoter to the GUS gene.

The CAB promoter fragment can be cloned into the GUS expression vectors pTAK1/3 (17) by insertion into the XbaI-BamH1 sites found in the polylinker region of these vectors.

2.6 Fusion of the CAB promoter to the B.t. gene.

The CAB promoter isolated on an Xbal-Sau3A fragment is cloned into pUC19 cut with BamH1 and Xba1. This yields pTCAB1. The B.t. gene is excised from pJRD2 by cutting it with HindIII and partially cutting it with ClaI. The resulting 2.4 kb fragment containing the B.t. gene and the nos terminator is made blunt-ended using Klenow polymerase fragment A. This fragment is cloned into pTCAB1 cut with SmaI to yield pTCBT1. This transcriptional fusion is a direct DNA transformation vector. In order to transfer it to Bin 19 in use for Agrobacterium mediated transformation, pTCBT1 is cut with EcoRI and HindIII. The resulting fragment is cloned into Bin 19.

EXAMPLE 3

Transformation of tobacco.

3.1 Transformation of tobacco plants with pCG1.

The vector pCG1 described in 2.2 above (Figure 4), based on the binary plant transformation vector Bin19, was transferred to Agrobacteria by triparental mating. A culture of these bacteria is then used to transfer pCG1 DNA to tobacco plants using standard protocols (18). Transformed plants were generated after selection on kanamycin-containing media (using the method described in EPA 270248). Plants transformed with the GUS expression module were assayed for GUS activity at room temperature using standard protocols.

Results are shown in Table 3.1 below. They indicate that the maize CAB gene promoter drives expression of foreign genes in transgenic plants.

## TABLE 3.1

| Plant | Fu*/hr/$\mu$g protein | Plant | Fu*/hr/$\mu$g protein |
|---|---|---|---|
| 1 | 19.4 | 36 | 5.2 |
| 2 | 0 | 37 | 0 |
| 3 | 0 | 38 | 4.9 |
| 4 | 83.1 | 39 | 44.0 |
| 6 | 0 | 40 | 54.0 |
| 7 | 0 | 41 | 24.3 |
| 8 | 0 | 42 | 0 |
| 9 | 0 | 43 | 0 |
| 11 | 0 | 44 | 1.7 |
| 13 | 8.4 | 45 | 0 |
| 14 | 17.1 | 46 | 12.9 |
| 16 | 10.3 | 47 | 6.0 |
| 17 | 22.8 | 48 | 9.7 |
| 18 | 24.5 | 49 | 30.7 |

**TABLE 3.1** (continued)

| Plant | Fu*/hr/μg protein | Plant | Fu*/hr/μg protein |
|-------|-------|-------|-------|
| 19 | 0 | 50 | 0 |
| 20 | 0 | 51 | 7.3 |
| 21 | 0 | 52 | 0 |
| 22 | 20.6 | 53 | 0 |
| 23 | 11.0 | 54 | 0 |
| 24 | 0 | 55 | 140.0 |
| 25 | 16.6 | 56 | 0 |
| 26 | 0 | 57 | 29.0 |
| 27 | 54.4 | 58 | 0 |
| 28 | 89.0 | 59 | 46.0 |
| 29 | 38.0 | 60 | 2.6 |
| 30 | 1.4 | 61 | 0 |
| 31 | 5.6 | 62 | 6.0 |
| 33 | 0 | 63 | 6.6 |
| 34 | 13.6 | 64 | 3.2 |
| 35 | 0 | 65 | 15.7 |
| Control Plants | | | |
| Gus +ve | 32.1 | | |
| Gus −ve | 0 | | |

\* **Fluorescence units**

3.2 Transformation of pCBT2 into tobacco plants.

The transformation of tobacco plants, as in 3.1, is repeated using the vector pCBT2 from Example 2.3. Plants transformed with B.t. expression cassettes are tested for their ability to withstand infection by a test organism, Heliothis virescens. Six first instar larvae are placed on transformed plants. Leaf area eaten is assessed using a semi-quantitative test.

A first bioassay was carried out on 24 plants using six Heliothis larvae for infestation of each plant. The results are shown in Table 3.2 below and show that transformant plants appear to be damaged to a lesser extent than control plants of similar size. In plants Nos. 21 and 31, for example, damage is only 33% of that of the control.

TABLE 3.2

| PLANT | % LEAF AREA EATEN | AVERAGE | S.D | TOTAL LEAF AREA ($mm^2$) | AVERAGE | S.D | LIVE LARVAE | LARVAL MASS | AVER |
|---|---|---|---|---|---|---|---|---|---|
| 2 | 34 | | | 6900 | | | 2 | 0.00195 | |
| 5 | 41 | | | 3900 | | | 3 | 0.00787 | |
| 8 | 50 | | | 4600 | | | 3 | 0.01347 | |
| 11 | 61 | | | 5300 | | | 5 | 0.01784 | |
| 12 | 68 | | | 5800 | | | 6 | 0.01180 | |
| 15 | 55 | | | 4800 | | | 5 | 0.00784 | |
| 16 | 32 | | | 8800 | | | 4 | 0.01070 | |
| 21 | 21 | | | 5700 | | | 2 | 0.00340 | |
| 26 | 34 | | | 4400 | | | 4 | 0.00715 | |
| 27 | 24 | | | 5200 | | | 4 | 0.00800 | |
| 30 | 42 | | | 5400 | | | 4 | 0.01363 | |
| 31 | 22 | 77.2 | 18.6 | 5800 | 5550 | 1288 | 5 | 0.00378 | 0.00 |
| 3 | 22 | | | 8000 | | | 6 | 0.00337 | |
| 4 | 25 | | | 7100 | | | 6 | 0.00870 | |
| 9 | 34 | | | 6450 | | | 5 | 0.01198 | |
| 10 | 30 | | | 8000 | | | 6 | 0.01085 | |
| 13 | 18 | | | 6100 | | | 4 | 0.00393 | |
| 14 | 23 | | | 7400 | | | 3 | 0.01290 | |
| 17 | 28 | | | 4200 | | | 4 | 0.00725 | |
| 19 | 18 | | | 8700 | | | 6 | 0.00453 | |
| 20 | 29 | | | 6300 | | | 4 | 0.01223 | |
| 24 | 27 | | | 5900 | | | 6 | 0.00468 | |
| 32 | 32 | | | 7100 | | | 5 | 0.00912 | |
| 36 | 26 | 26 | 5.1 | 6300 | 6796 | 1195 | 5 | 0.00934 | 0.00 |

EP 0 353 908 B1

TABLE 3.2 (continued)

| PLANT | % LEAF AREA EATEN | AVERAGE | S.D | TOTAL LEAF AREA (mm²) | AVERAGE | S.D | LIVE LARVAE | LARVAL MASS | AVER |
|---|---|---|---|---|---|---|---|---|---|
| CONTROLS | | | | | | | | | |
| A | 63 | | | 8300 | | | 5 | 0.01504 | |
| B | 16 | | | 6800 | | | 3 | 0.00147 | |
| C | 61 | | | 5700 | | | 5 | 0.00936 | |
| D | 37 | | | 6300 | | | 2 | 0.01320 | |
| E | 69 | | | 5600 | | | 4 | 0.01123 | |
| F | 64 | | | 5600 | | | 2 | 0.01540 | |
| G | 28 | | | 12100 | | | 2 | 0.01930 | |
| H | 11 | | | 12300 | | | 0 | | |
| I | 62 | | | 5800 | | | 4 | 0.01360 | |
| J | 14 | | | 9300 | | | 1 | 0.01710 | |
| K | 27 | | | 13000 | | | 4 | 0.01313 | |
| L | 26 | | | 9700 | | | 2 | 0.00915 | |
| M | 23 | | | 10000 | | | 1 | 0.01240 | |
| N | 22 | | | 10900 | | | 3 | 0.01010 | |
| O | 18 | | | 8400 | | | 0 | | |
| P | 30 | | | 6900 | | | 0 | | |
| Q | 54 | 36.8 | 19.9 | 5200 | 8347 | 2628 | 4 | 0.01403 | 0.01 |

The proportion of plants showing some degree of protection is unexpectedly high, given the number of plants tested. From previous experience, it had been expected that only about 14 of the 24 plants would have shown any significant expression of the δ-endotoxin. It was also expected that the occurrence of a dramatically increased level of protection would be about 1 in 60 plants and, indeed, none of the transformed plants tested showed a dramatic increase in protection from attack.

### 3.3 Transformation of tobacco protoplasts.

Tobacco leaf mesophyll protoplasts were prepared following published methods (19), and used in PEG-mediated DNA uptake experiments with the plasmid pCG2 from Example 2.2. Expression of CAB-promoter driven bacterial marker gene (GUS) was measured using standard methods. The results indicate that the maize CAB promoter drives the expression of the GUS gene very effectively in this system (Table 3.3).

TABLE 3.3

| Construct | GUS activity (units) | Plasmid |
|---|---|---|
| no rDNA | - | - |
| CaMV35s-GUS | 4.6 | pGS203 |
| CAB-GUS (light) | 88.8 | pCG2 |
| CAB-GUS (dark) | 14.5 | pCG2 |

EXAMPLE 4 Analysis of transformed tobacco plants.

### 4.1 Southern analysis

DNA is isolated from transformed plants produced in Example 3 and the presence of the transferred gene constructs demonstrated Southern hybridisation.

Plasmids and constructs referred to in the present specification were deposited in Escherichia coli, strain TG2 hosts, at the National Collections of Industrial and Marine Bacteria (NCIMB) Torry Research Station, PO Box 31, 135 Abbey Road, Aberdeen AB9 8DG, Scotland under the following references and on the dates stated:

| Plasmid | Example or Experiment | NCIMB No. | Date |
|---|---|---|---|
| pCAB48.1 | 2.1 | 40036 | 22 July 1988 |
| pCG2 | 2.2 | 40037 | 22 July 1988 |
| pCBT1 | 2.6 | 40038 | 22 July 1988 |
| pTAK1 | 2.5 | 12353 | 11 Nov. 1986 |
| pTAK3 | 2.5 | 12355 | 11 Nov. 1986 |

References

1. Boardman et al Curr. Top. Bionerg. 8, 35-109 (1978)
2. Kirk et al, The Plastids, Elsevier 1-90 (1978)
3. Ellis Ann. Rev. Plant Physiol. 32 11-137 (1981)
4. Thornber Ann. Rev. Plant Physiol. 26, 127-158 (1975)
5. Nelson et al. J. Cell Biol. 98, 558-564 (1984)
6. Lamppa et al Mol. Cell. Biol. 5, 1370-1378 (1985)
7. Coruzzi et al EMBO J. 3, 1671-1679 (1984)
8. Greenland et al Planta 170, 99-110 (1987)
9. Pichersky et al Plant Mol Biol 9, 205-216 (1987)
10. Castresana et al Plant Mol Biol 10, 117-126 (1987)
11. Lamppa et al Nature 316, 750-752 (1985)
12. Simpson et al. Nature 323, 551-554 (1986)
13. Nagy et al. EMBO J. 6, 2537-2542 (1987)
14. Murray and Thompson NAR 8, 4321-4325 (1980)
15. Maniatis et al Cloning Manual (1982)
16. Sauger et al PNAS 74, 5463-5467 (1977)
17. Jefferson et al EMBO J (1987)
18. Bevan NAR 12, 8711-8721 (1984)

19. Negrutiu et al Plant Mol Biol 8, 363-373 (1987)
20. EPA 270248 (ICI)
The disclosure of the above documents is incorporated herein by reference.

**Claims**

1. A DNA construct comprising the promoter from a chlorophyll a/b binding (CAB) protein gene derived from the plant Zea mays, said promoter being operatively linked to exogenous DNA coding for RNA exogenous to Zea mays, whereby the construct expresses the exogenous RNA only under control of the promoter.

2. A construct as claimed in claim 1 in which the exogenous DNA encodes a $\delta$-endotoxin of Bacillus thuringiensis.

3. A construct as claimed in claim 1 in which the exogenous DNA encodes for antisense mRNA having a complementary sequence to mRNA expressed by an endogenous plant gene.

4. A vector containing a DNA construct as claimed in claim 1 or claim 2 or claim 3.

5. A vector as claimed in claim 4 comprising a bacteriophage or a plasmid.

6. A plant genome having stably incorporated therein a recombinant DNA comprising the promoter from a CAB protein gene derived from the Zea mays, operatively linked to DNA coding for RNA exogenous to Zea mays, whereby the RNA is expressed only under control of the promoter.

7. A plant having stably incorporated in its genome a recombinant DNA comprising the promoter from a CAB protein gene derived from Zea mays, operatively linked to DNA coding for the expression of RNA exogenous to Zea mays, whereby the RNA is expressed only in plant green tissue.

8. The maize CAB protein gene promoter comprising the fragment, derived from Zea mays, of the base sequence set forth in Figure 2.

9. A DNA construct comprising a CAB protein gene promoter fused to a $\delta$-endotoxin gene of Bacillus thuringiensis strain HD-73, said construct being present in plasmid pCBT1 (NCIMB 40038).

**Patentansprüche**

1. DNA-Konstruktion, die den Promotor von einem Chlorophyll a/b-Bindungs (CAB)-Protein-Gen, das von der Pflanze Zea mays stammt, enthält, wobei der Promotor operativ an exogene DNA gebunden ist, die für Zea mays exogene RNA codiert, wodurch die Konstruktion die exogene RNA nur unter der Kontrolle des Promotors exprimiert.

2. Konstruktion nach Anspruch 1, wobei die exogene DNA ein $\delta$-Endotoxin von Bacillus thuringiensis codiert.

3. Konstruktion nach Anspruch 1, wobei die exogene DNA Anti-sense-mRNA mit komplementärer Sequenz zu durch ein endogenes Pflanzen-Gen experimierter mRNA codiert.

4. Vektor, der eine DNA-Konstruktion nach Anspruch 1 oder Anspruch 2 oder Anspruch 3 enthält.

5. Vektor nach Anspruch 4, der einen Bacteriophagen oder ein Plasmid umfaßt.

6. Pflanzengenom mit einer stabil darin einverleibten rekombinanten DNA, die den Promotor eines von Zea mays stammenden CAB-Protein-Gens enthält, der operativ an für Zea mays exogene RNA codierende DNA gebunden ist, wodurch die RNA nur unter der Kontrolle des Promotors exprimiert wird.

7. Pflanze, die in ihrem Genom eine stabil einverleibte rekombinante DNA enthält, die den Promotor eines von Zea mays stammenden CAB-Protein-Gens enthält, der operativ an DNA gebunden ist, welche die Expression von für Zea mays exogene RNA codiert, wodurch die RNA nur im grünen Pflanzengewebe exprimiert wird.

8.  Mais-CAB-Protein-Genpromotor, der das aus Zea mays stammende Fragment mit der in Figur 2 angegebenen Basensequenz enthält.

9.  DNA-Konstruktion, die einen an ein δ-Endotoxin-Gen des Bacillus thuringiensis-Stamms HD-73 fusionierten CAB-Protein-Genpromotor enthält, wobei sich die Konstruktion im Plasmid pCBT1 (NCIMB 40038) befindet.

**Revendications**

1.  Produit d'assemblage d'ADN comprenant le promoteur provenant d'un gène de protéine de liaison a/b de chlorophylle (CAB) issu de la plante Zea mays, ledit promoteur étant lié de manière fonctionnelle à un ADN exogène codant pour un ARN exogène à Zea mays, le produit d'assemblage exprimant ainsi l'ARN exogène seulement sous le contrôle du promoteur.

2.  Produit d'assemblage suivant la revendication 1, dans lequel l'ADN exogène code pour une δ-endotoxine de Bacillus thuringiensis.

3.  Produit d'assemblage suivant la revendication 1, dans lequel l'ADN exogène code pour un ARNm antisens ayant une séquence complémentaire de celle d'un ARNm exprimé par un gène végétal endogène.

4.  Vecteur contenant un produit d'assemblage d'ADN suivant la revendication 1 ou la revendication 2 ou bien la revendication 3.

5.  Vecteur suivant la revendication 4, comprenant un bactériophage ou un plasmide.

6.  Génome de végétal ayant incorporé de manière stable un ADN recombinant comprenant le promoteur provenant d'un gène de protéine CAB issu de la plante Zea mays, lié de manière fonctionnelle à un ADN codant pour un ARN exogène à Zea mays, l'ARN étant ainsi exprimé seulement sous le contrôle du promoteur.

7.  Plante ayant incorporé de manière stable dans son génome un ADN recombinant comprenant le promoteur provenant d'un gène de protéine CAB issu de Zea mays, lié de manière fonctionnelle à un ADN codant pour l'expression d'un ARN exogène à Zea mays, l'ARN étant ainsi exprimé seulement dans les tissus verts de la plante.

8.  Promoteur de gène de protéine CAB de maïs comprenant le fragment, issu de Zea mays, ayant la séquence de base indiquée sur la figure 2.

9.  Produit d'assemblage d'ADN comprenant un promoteur de gène de protéine CAB fusionné à un gène de δ-endotoxine de la souche HD-73 de Bacillus thuringiensis, ledit produit d'assemblage étant présent dans le plasmide pCBT1 (NCIMB 40038).

Figure 1.

Restriction maps of gCAB 48, pCAB 48 and pCAB 48.1

EP 0 353 908 B1

FIGURE 2

SEQUENCE OF THE XbaI - NcoI  CAB PROMOTER FRAGMENT.

```
                10        20        30        40        50
       XbaI     :         :         .         :         :          :
     1 GTCTAGATATGTTTCTCTTTTGGTTCTTGAGTTGACAAGTGGCATGCTAT
    51 TTTTGCTCATGTGAGAAAAAATATAGCACCGTTTGTTTCGTTGAAATTGA
   101 ATTGCATCCCAATAATCATAATTTAGACATAAACCAACTAAGTTAATATA
   151 TTTGTATATGTAAATATGTTATCCTAAATTATAATTATATGAGAGAGATA
   201 GTTATACATTACAATTATGATATAGAGAAGCAAATAGAAGAGTGTGCTAT
   251 AAGTTGTACATTGTAAAAGTAGTATGCAAATTTATAGAATTAATTTTTAT
   301 CTTTCACCTCATAAATTTAAGATACACTTATATATAAACTTTGAAAAGTT
   351 GTAAAATATCATATTCTAAAAAAAATAGACTATTCTATTAGTTAGATTCC
   401 AATTCCTTAAAATAAAAGAAAATAAATGGGCTTAAGGGCTAGTTTGGTGG
   451 GCAGGTGGAGGCGATCAATGGCGGATGAATCCTCTCGATATTCAATTGAG
   501 GGGGTTCATCCCATGAATCTCTCTATTTACATGTCCACCAAACAAGATTA
   551 AATGCAATGATACATAGCTTTTGTGCATATTTTTTTTTAACGGAGATGCA
   601 AAACAGTATACAAGATGTGTGTATGTTCCCATGCGACCCATCTTGTATCC
   651 AGTTCATGCGTCTGGTGGTTGAACCAGAAAGATACACATGATCCATTTCG
   701 ATGAATCAACACAAAAGACTTCATTGATTATGCATTTGACAAATAGCAAG
   751 AAGCGCGGGGCAACAAAGCCCATCCTATCTCATCCATTTTTTCCCACGAT
   801 GGCAAGTGGCAGCTCCTGATTAGCTACGCCCATTCCTATGCTATGTGGCA
   851 CACCCCAGGATTCTTGTGTGATAGGCCATTGGGGCCACGAGGAGCCACGT

                                         _____   _____
   901 CAGACGCCAAGCCACCCCGGCGAGACCAACCAGCCAATCGCAGTTCAGGA

                                     _____
   951 AAAGATGACCTGCTATCCAAACCCAACTGTATAAAAGGCAGCTGCTGTGT
          *  *  .*    *
  1001 TCTGTTATGACACAGCCATCACACGCATACTGCATACACAACACAGAGCA
                              M  A  A  A  T  M
  1051 TCAGAAGGAGCTACGATCTGATCGACATGGCTGCCGCCACCATGG
                                               NcoI
```

_____ = CAT and TATA sequences.

  *  = Possible transcription start sites.

# STRUCTURE OF pCAB 48.1

tsp = Approximate position of transcript start

■■■■ = CAB coding region

——→ = Extent of sequence obtained from each clone

Figure 3

EP 0 353 908 B1

Figure 4

# CAB-GUS CONSTRUCTION

EP 0 353 908 B1

FIG 5

```
       GCCATG GCTATCGAAG TATTAGGTGG AGAAAGAATA GAAACTGGTT ACACCCCAAT CGATATCATC ATCATCAA
ACGTCGGTAC CGATAGCTTC ATAATCCACC TCTTTCTTAT CTTTGACCAA TGTGGGGTTA GCTATAGTAG TAGTAGTTTC GA
            10         20         30         40         50         60         70         80
```

17

Figure 6

## CAB-Bt HD-73 CONSTRUCTION